# EUROPEAN PATENT APPLICATION

(11) **EP 1 849 785 A1**
(43) Date of publication of application: **31.10.2007**
(21) Application number: 06380099.9
(22) Date of filing: 28.04.2006
(51) Int. Cl.: C07D 417/12, A61K 31/426, A61K 31/427, A61P 25/28

(54) **N-(2-Thiazolyl)-amide derivatives as GSK-3 inhibitors**

(71) Applicant: Neuropharma, S.A., 28760 Madrid (ES)
(72) Inventor: Martinez Gil, Ana, 28004 Madrid (ES); Castro Morera, Ana, 28004 Madrid (ES); Medina Padilla, Miguel, 28029 Madrid (ES); Martin Aparicio, Ester, 28411 Moralzarzal (ES); Alonso Cascon, Mercedes, 28036 Madrid (ES); Fuertes Huerta, Ana, 28003 Madrid (ES); Navarro Rico, Maria Luisa, 28014 Madrid (ES); Perez Puerto, Maria Jose, 28008 Madrid (ES); Del Monte Millan, Maria, 28032 Madrid (ES)
(74) Representative: ABG Patentes, S.L.

(57) **Abstract**

The present invention relates to the use of N-(2-thiazolyl)-amide derivatives of formula (I) 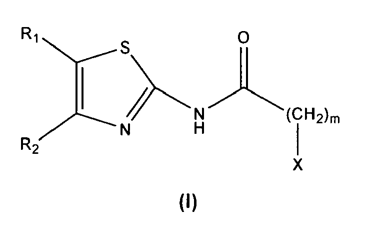 wherein
X is selected from the group consisting of:
- an indol of formula (A) or (B), bonded respectively at positions 2 or 3: 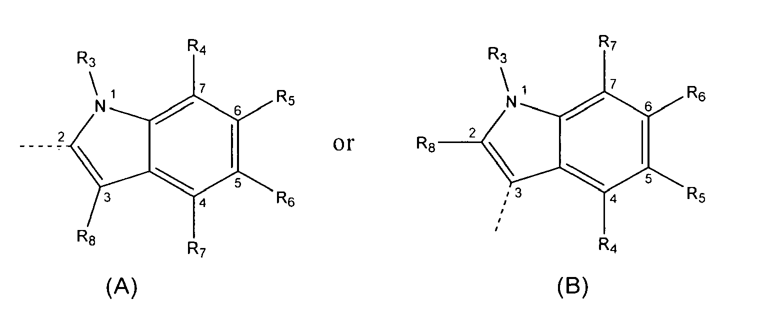 - an indazol of formula (C), bonded at position 3: 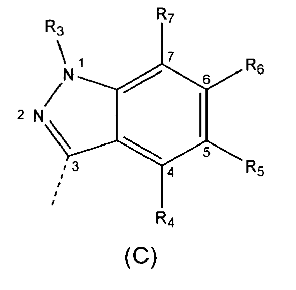 - a pyridin, bonded at any positions 2 to 6; and
- a phenyl
for the treatment and/or prophylaxis of a disease in which glycogen synthase kinase 3 (GSK-3) is involved, especially neurodegenerative diseases, such as Alzheimer's disease, or non-insulin dependent diabetes mellitus.

## Description

### FIELD OF THE INVENTION

The present invention relates to the use of N-(2-thiazolyl)-amide derivatives for the treatment and/or prophylaxis of a disease in which glycogen synthase kinase 3 (GSK-3) is involved, particularly neurodegenerative diseases, such as Alzheimer's disease, or non-insulin dependent diabetes mellitus. Additionally, there is provided new GSK-3 inhibitors, a process for preparing such compounds and pharmaceutical compositions comprising them.

### BACKGROUND OF THE INVENTION

The search for new therapeutic agents has been greatly aided in recent years by better understanding the structure of enzymes and other biomolecules associated with target diseases. One important class of enzymes that has been the subject of extensive study is the protein kinases. Many diseases are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease or hormone-related diseases. Accordingly, there has been a substantial effort in medicinal chemistry to find protein kinase inhibitors that are effective as therapeutic agents.

Glycogen synthase kinase-3 (GSK-3) is a serine/threonine protein kinase comprised of α and β isoforms that are each encoded by distinct genes (Coghlan et al., Chemistry & Biology, 7, 793-803 (2000); Kim and Kimmel, Curr. Opinion Genetics Dev., 10, 508-514 (2000)). The threonine/serine kinase glycogen synthase kinase-3 (GSK-3) fulfills a pivotal role in various receptor-linked signalling pathways (Doble, BW, Woodgett, JR J.Cell Sci. 2003, 116:1175-1186). Dysregulation within these pathways is considered a crucial event in the development of several prevalent human disorders, such as type II diabetes (Kaidanovich O, Eldar-Finkelman H, Expert Opin. Ther. Targets, 2002, 6:555-561), Alzheimer's disease (Grimes CA, Jope RS, Prog.Neurobiol. 2001, 65:391-426), CNS disorders such as manic depressive disorder and neurodegenerative diseases, and chronic inflammatory disorders (Hoeflich KP, Luo J, Rubie EA, Tsao MS, Jin O, Woodgett J, Nature 2000, 406:86-90). These diseases may be caused by, or result in, the abnormal operation of certain cell signalling pathways in which GSK-3 plays a role.

GSK-3 has been found to phosphorylate and modulate the activity of a number of regulatory proteins. These proteins include glycogen synthase which is the rate limiting enzyme necessary for glycogen synthesis, the microtubule associated protein Tau, the gene transcription factor β-catenin, the translation initiation factor e1 F2B, as well as ATP citrate lyase, axin, heat shock factor-1, c-Jun, c-Myc, c-Myb, CREB, and CEPBα. These diverse protein targets implicate GSK-3 in many aspects of cellular metabolism, proliferation, differentiation and development.

Currently, inhibition of GSK-3 may represent a viable strategy to develop novel medicinal entities for the treatment of such unmet diseases (Martinez A, Castro A, Dorronsoro I, Alonso M, Med. Res. Rev., 2002, 22:373-384) through insulin mimicry, tau dephosphorylation and amyloid processing, or transcriptional modulation respectively.

The neurotoxic effect of soluble and deposited amyloid β peptides (Aβ) is a characteristic pathology in the brains of patients with Alzheimer's Disease (AD). Both in vitro and in vivo studies suggest that Aβ peptides induce loss of effectiveness of the Wnt signaling pathway, and this mechanism seems to be mediated by a destabilization of the endogenous levels of β-catenin *(*Activation of Wnt signaling rescues neurodegeneration and behavioural impairments induced by beta-amyloid fibrils, de Ferrari et al, Mol. Psychiatry. 2003;8(2):195-208). Activation of Wnt signaling pathway by lithium or Wnt ligands in AD cellular and animal experimental models, diminishes the neurotoxic effect of Aβ by restoring the normal levels of β-catenin and the expression of certain Wnt-target survival genes, such as bcl-2. Disorders in components of the Wnt pathway would trigger some events which could lead to the onset and development of AD *(*Signal transduction during amyloid-beta-peptide neurotoxicity: role in Alzheimer disease, Fuentealba et al., Brain Res. Rev. 2004;47(1-3):275-89).

The presence of neurofibrillary tangles in neurons of cerebral cortex is another abnormality which occurs in the brain of AD patients, and hyperphosporylated tau protein seems to be a main component of these neuronal deposits *(*Neurofibrillary tangles of Alzheimer disease share antigenic determinants with the axonal microtubule-associated protein tau, Wood JG et al., Proc. Natl. Acad. Sci. USA. 1986;83(11):4040-3). Tau is a set of six protein isoforms associated to the microtubules which modulates the functions of these cellular structures in the axonal compartments of neurons. Tau can be phosphorylated by different microtubule-associated kinases but GSK3β and cdk5 are the ones whose effects contribute most to the formation of neurofibrillary tangles *(*Phosphorylation of human tau protein by microtubule-associated kinases: GSK3β and cdk5 are key participants, Flaherty et al., J. Neurosci. Res. 2000;62:463-472). Indeed, the activity of GSK-3 seems to trigger the assembling of the filaments that form the neurofibrillary tangles *(*Glycogen synthase kinase 3 alteration in Alzheimer disease is related to neurofibrillary tangle formation, Baum et al., Mol. Chem. Neuropathol. 1996;29 (2-3):253-61). Thus, phosphorylation of protein tau is another key role of GSK-3 that has an influence in the pathology of AD.

These facts related to the physiological events occurring in AD support that GSK-3 may be an important target for a treatment of this disease, not only for its modulation in the Wnt pathway, but also for its influence in the formation of Aβ neurofibrillary tangles.

Another pathology wherein Wnt signaling is involved is Parkinson's Disease. A physiological characteristic of this illness is the decrease of neurons which produce dopamine, although the reasons that provoke this event are not completely known. Wnt proteins have an important role in the differentiation process of these nerve cells. Normalization of β-catenin levels by GSK-3 inhibitors leads to an increase of the differentiation of dopaminergic neurons *(*GSK-3beta inhibition/beta-catenin stabilization in ventral midbrain precursors increases differentiation into dopamine neurons, Castelo- Branco et al., J Cell Sci. 2004;117(Pt 24):5731-7).

GSK-3 also plays an important role modulating the cellular action of insulin through the phosphorylation of glycogen synthase, the enzyme that catalyzes the condensation of glucose monomers to form glycogen. The phosphorylation of glycogen synthase by GSK-3 and other kinases leads to its inactivation and this event attenuates the effect of insulin in cells. Indeed, several selective GSK-3 inhibitors have been proven to mimic insulin action in vitro and in vivo models *(*Insulin mimetic action of synthetic phosphorylated peptide inhibitors of glycogen synthase kinase-3, Plotkin et al., Pharmacol Exp Ther. 2003;305(3):974-80). According to these experimental results, inhibition of GSK-3 may have a therapeutic effect in the treatment of insulin resistance and type 2 diabetes.

In view of the above, GSK3 inhibitors are a potential treatment of Alzheimer's Disease, Parkinson's Disease, diabetes and some other diseases.

Tau is a family of proteins whose main role in cells is promoting microtubules stability. Microtubules are the main component of the cytoskeleton, an important cellular organelle, especially for neurons. The major role of the cytoskeleton in neurons is providing the structural support to form the axonal and the somatodendritic compartments, which are part of a neuronal network essential for the correct function of the CNS. The cytoskeleton is a critical element for the survival of neurons and many neuronal and neurodegenerative diseases are characterized by abnormalities in it. Therefore, tau and other proteins involved in the cytoskeleton structure may be promising targets for the treatment of many neuronal and neurodegenerative disorders.

The tau isoforms come from an alternative mRNA splicing of a single gene, which results in six different peptidic chains with molecular weights between 50 and 70 kDa. Tau proteins are highly expressed in the central and peripheral nervous system, and they are especially abundant in the axons of neurons, where they contribute to the organisation and integrity of the synaptic connections in the CNS.

Some studies (Brandt & Lee, J Biol. Chem. 1993, 268, 3414-3419 and Trinczek et al., Mol. Biol. Cell. 1995, 6, 1887-1902) have demonstrated that tau is capable of promoting microtubules nucleation, growth and assembling. These functions of tau are regulated by phosphorilation / dephosphorilation processes which occur in multiple sites of its peptidic chain. Many kinases are capable of phosphorylating these sites in vitro, although there are fewer kinases capable of doing it in vivo. In normal physiological conditions, there is a balance between phosphorylated and dephosphorylated tau that regulates its binding to microtubules and to other proteins. However, some pathological events may disrupt this balance, eliminating the interactions between tau and microtubules and disassembling both cytoeskeleton elements. Phosphorylations in other sites of tau induce an increase of tau-tau interactions and a subsequent formation of tau oligomers, which finally aggregate into neurofibrillary tangles (NFTs). All these changes provoke the destruction of the microtubule transport system along the axons to the synapses, causing impairment of neuronal functions and eventually cell death.

Thus, disregulation of tau has been thought to be the hallmark of many neurological disorders, commonly known as tauopathies, which are characterized by an abnormal accumulation of tau filaments in the brain. Some remarkable tauopathies are, among others, Alzheimer's disease, Gerstmann-Sträussler-Scheinker disease, Pick's disease, amiotrophic lateral sclerosis (ALS), Creutzfeld-Jakob disease, Down's syndrome or prion protein cerebral amyloid angiopathy.

Many current researches are focused on the relationship between disregulation of tau and accumulation of amyloid plaques, the other main pathological hallmark of Alzheimer's disease. Some authors (Price et al, Annu. Rev. Genet, 1998, 32, 461-493 and Selkoe, Trends Cell Biol. 1998, 8, 447-453) suggest that amyloid pathology occurs upstream of tau pathology, although the related mechanism have not been clearly explained yet. It is thought that deposition of fibrillar amyloid beta induces the phosphorylation of tau which later provokes the neuronal degeneration.

In the light of state of the art and taking into account that GSK-3 enzyme as well as tau protein have a direct implication in a series of important human diseases and disorders, especially neuronal and neurodegenerative disorders, there is a need for finding effective inhibitors of said enzyme and of tau protein phosphorylation, in order to obtain effective medicaments for the treatment of such diseases and disorders.

### SUMMARY OF THE INVENTION

The present invention provides a family of compounds, namely N-(2-thuazolyl)-amide derivatives, defined by formula (I) as detailed below, displaying an inhibitory effect on GSK-3. They may thus be useful for the treatment of diseases and conditions wherein GSK-3 plays a role, especially neuronal and neurodegenerative diseases and conditions. Many of the compounds additionally show an inhibitory effect on tau protein phosphorylation, which also plays an important role in many neurodegenerative diseases, so the compounds of formula (I) may even have a dual role for treating or preventing neuronal and neurodegenerative diseases.

Accordingly, in a first aspect the present invention provides the use of a compound of formula (I): 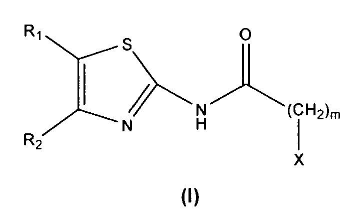 wherein
R₁ and R₂ are independently selected from H, -NO₂, halogen, -NH₂, -CF₃, C₁-C₆ linear alkyl and -CN;
m is an integer from 0 to 6,
X is selected from the group consisting of:
- an indol of formula (A) or (B), bonded respectively at positions 2 or 3: 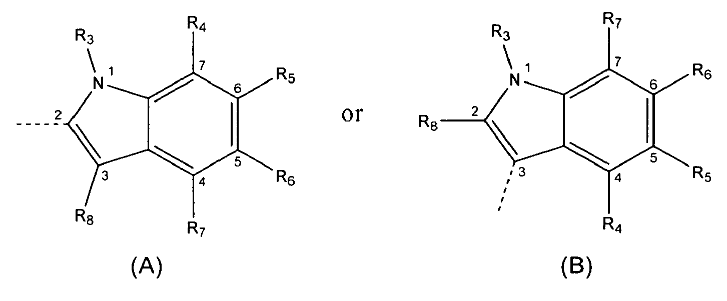
- an indazol of formula (C), bonded at position 3: 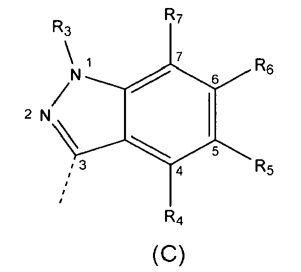
- a pyridin, bonded at any positions 2 to 6; and
- a phenyl
wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl,
or any pharmaceutically acceptable salts, solvates and prodrugs thereof, in the preparation of a medicament for the treatment or profilaxis of a disease or condition mediated by GSK-3.

The compounds of formula (I) may be used in biological assays wherein GSK-3 activity needs to be modulated. Therefore, in another aspect, the invention refers to the use of a compound of formula (I) as defined above, or any salt or solvate thereof, as reactive for modulating GSK-3 in biological assays, preferably as a reactive for inhibiting GSK-3 activity.

A further aspect of the invention refers to a method for the treatment of a disease in which GSK-3 is involved, comprising administering to a patient in need of such treatment a therapeutically effective amount of at least one compound of general formula (I) or a pharmaceutical composition thereof.

An additional aspect of the invention is a novel compound of formula (I): 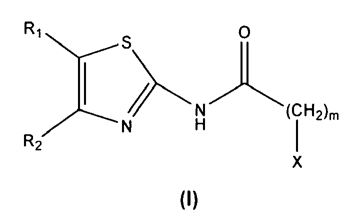 wherein:
R₁ and R₂ are independently selected from H, -NO₂, halogen, -NH₂, -CF₃, and -CN;
   with the proviso that at least one of R₁ and R₂ is different from H;
   m is an integer from 0 to 6,
X is selected from the group consisting of:
   - an indol of formula (A) or (B), bonded respectively at positions 2 or 3: 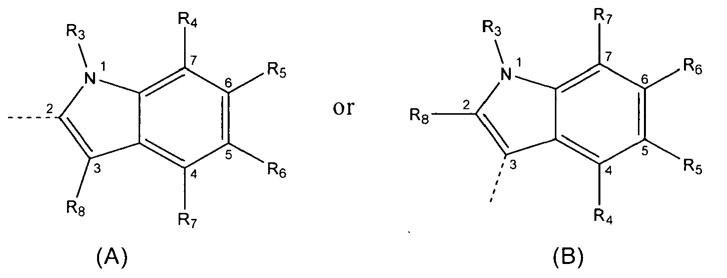
   - an indazol of formula (C), bonded at position 3: 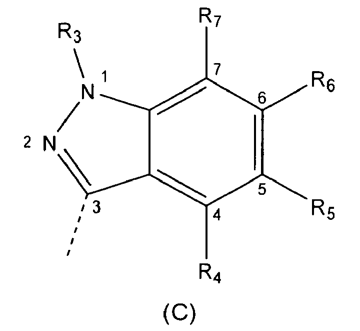 and
   - a pyridin, bonded at any positions 2 to 6;
      wherein
      R₃ is selected from H and C₁-C₆ linear alkyl;
      R₄, R₅, R₆ and R₇ are independently selected from H, C₁-C₆ alkyl, C₁-C₆ alkoxy and halogen;
      R₈ is selected from H and C₁-C₆ alkyl,
   or any pharmaceutically acceptable salts, solvates and prodrugs thereof.

According to a further aspect, the present invention is related to a novel compound of formula (I), for use as a medicament.

A further aspect of the present invention is a pharmaceutical composition, comprising at least one novel compound of formula (I), or any pharmaceutically acceptable salt, prodrug or solvate thereof, and a pharmaceutically acceptable carrier, adjuvant or vehicle.

Finally, another aspect of the invention relates to a process for the preparation of novel compounds of formula (I).

### DETAILED DESCRIPTION OF THE INVENTION

In the above definition of compounds of formula (I) the following terms have the meaning indicated:
"Alkyl" refers to a linear or branched hydrocarbon chain radical consisting of carbon and hydrogen atoms, containing no unsaturation, having one to eight carbon atoms, and which is attached to the rest of the molecule by a single bond, e. g., methyl, ethyl, n-propyl, i-propyl, n-butyl, t-butyl, n-pentyl, etc.
"Alkoxy" refers to a radical of the formula -ORₐ where Rₐ is an alkyl radical as defined above, e. g., methoxy, ethoxy, propoxy, etc.
"Halogen" refers to a chloro, bromo, fluoro, or iodo substituent.

In a first aspect the present invention provides the use of a compound of formula (I): 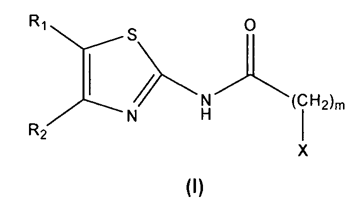 wherein
R₁ and R₂ are independently selected from H, -NO₂, halogen, -NH₂, -CF₃, C₁-C₆ linear alkyl and -CN;
m is an integer from 0 to 6,
X is selected from the group consisting of:
- an indol of formula (A) or (B), bonded respectively at positions 2 or 3: 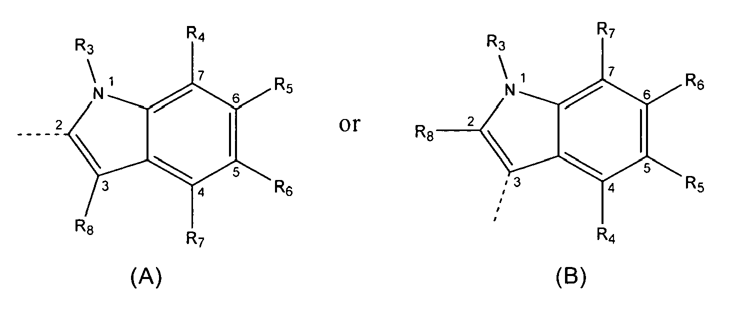
- an indazol of formula (C), bonded at position 3: 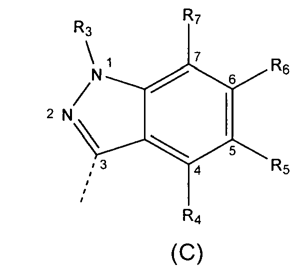
- a pyridin, bonded at any positions 2 to 6; and
- a phenyl
wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl,
or any pharmaceutically acceptable salts, solvates and prodrugs thereof, in the preparation of a medicament for the treatment or profilaxis of a disease or condition mediated by GSK-3.

Within the frame of the present invention, a disease or condition mediated by GSK-3 means any disease or condition in which GSK-3 is involved, preferably any disease or condition requiring GSK-3 inhibition. Such disease or condition includes, but is not limited to, any disease or condition selected from diabetes, conditions associated with diabetes, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, Gerstmann-Sträussler-Scheinker disease, Creutzfeld-Jakob disease, prion protein cerebral amyloid angiopathy, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, manic depressive disorder, promotion of functional recovery post stroke, cerebral bleeding (for example, due to solitary cerebral amyloid angiopathy), hair loss, obesity, atherosclerotic cardiovascular disease, hypertension, polycystic ovary syndrome, syndrome X, ischaemia, brain injury, especially traumatic brain injury, cancer, leukopenia, Down's syndrome, Lewy body disease, inflammation, chronic inflammatory diseases, cancer and hyperproliferative diseases as hyperplasias and immunodeficiency.

In a preferred embodiment, the disease or condition is selected from progressive supranuclear palsy, Pick's disease, corticobasal degeneration, frontotemporal dementia, Huntington's disease, amyotrophic lateral sclerosis, multiple sclerosis and neurotraumatic diseases such as acute stroke, epilepsy, mood disorders such as depression, schizophrenia and bipolar disorders, manic depressive disorder, promotion of functional recovery post stroke, cerebral bleeding (for example, due to solitary cerebral amyloid angiopathy), obesity, syndrome X, ischaemia, brain injury, especially traumatic brain injury, Down's syndrome, Lewy body disease, inflammation, chronic inflammatory diseases, cancer and hyperproliferative diseases as hyperplasias. More preferably, the disease or condition is selected from Alzheimer's disease, diabetes, Parkinson's disease, epilepsy and mood disorders.

Unless otherwise stated, the compounds of formula (I) used in the present invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates or prodrugs" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of the compounds of formula (I) are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The compounds of formula (I) used in the invention may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The compounds used in the invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

The compounds of formula (I) may be used in biological assays wherein GSK-3 activity needs to be modulated. Therefore, in another aspect, the invention refers to the use of a compound of formula (I) as defined above, or any salt or solvate thereof, as reactive for modulating GSK-3 in biological assays, preferably as a reactive for inhibiting GSK-3 activity.

A further aspect of the invention refers to a method for treating or preventing a disease, disorder or condition in which GSK-3 is involved, said method comprising administering to a patient in need of such treatment a therapeutically effective amount of at least one compound of general formula (I) or any salt or solvate thereof, or a pharmaceutical composition thereof.

Another aspect of the invention relates to a novel compound of formula (I): 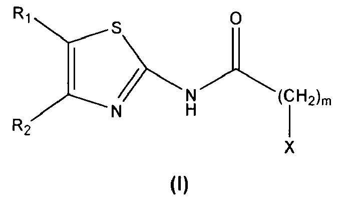 wherein:
R₁ and R₂ are independently selected from H, -NO₂, halogen, -NH₂, -CF₃, and -CN;
   with the proviso that at least one of R₁ and R₂ is different from H;
   m is an integer from 0 to 6,
X is selected from the group consisting of:
   - an indol of formula (A) or (B), bonded respectively at positions 2 or 3: 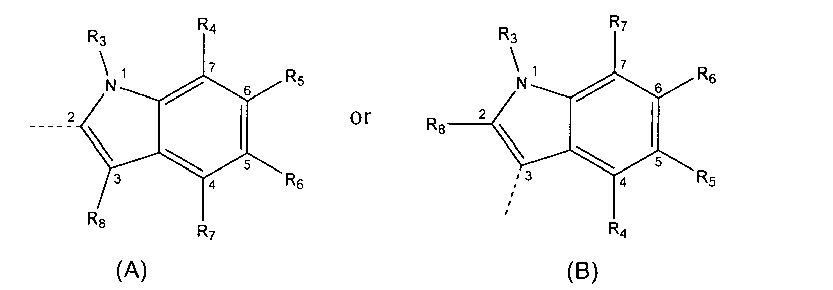
   - an indazol of formula (C), bonded at position 3: 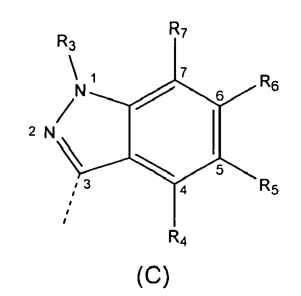
   - a pyridin, bonded at any positions 2 to 6;
wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl,
or any pharmaceutically acceptable salts, solvates and prodrugs thereof.

Preferred compounds are those wherein X is an indol of formula (A) or (B) as defined above, bonded at positions 2 or 3. Further preferred compounds are those
wherein X is an indazol of formula (C) as described above, bonded at position 3.

Other preferred compounds are those wherein one of R₁ and R₂ is H.

Preferably, one of R₁ or R₂ is -NO₂. Thus, more preferred compounds are those
wherein one of R₁ and R₂ is -NO₂ and the other is H. Even more preferred compounds are those wherein R₁ is -NO₂ and R₂ is H.

In a further preferred embodiment, R₃ is selected from H and methyl.

Further preferred compounds are those wherein R₄ to R₇ are independently selected from methoxy and H. Preferably, all of R₄ to R₇ are H.

Compounds wherein R₈ is selected from H and methyl are also preferred.

Preferably, m is selected from 0, 1 or 2, where m=0 means that the amide group is directly linked to X through the carbonyl group.

According to a preferred embodiment, the compound of formula (I) is selected from the following compounds:

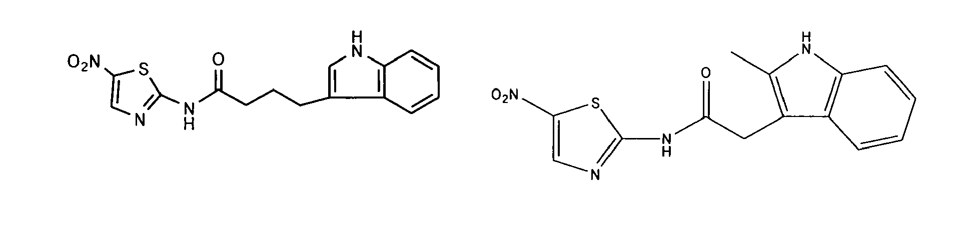

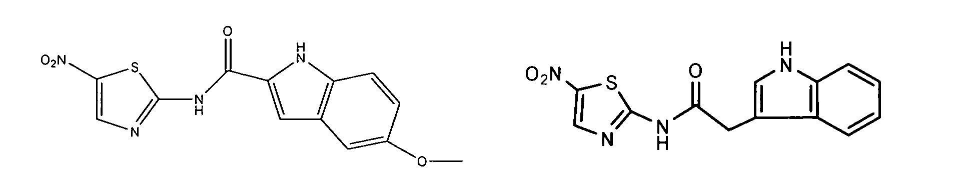

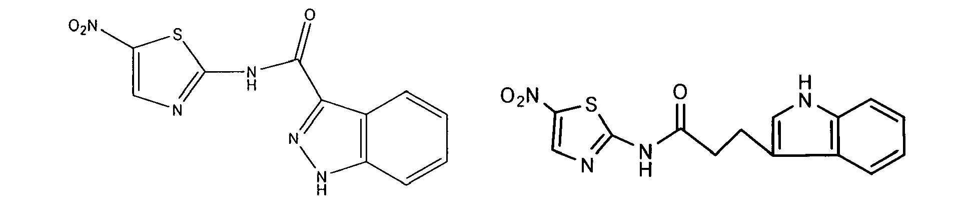

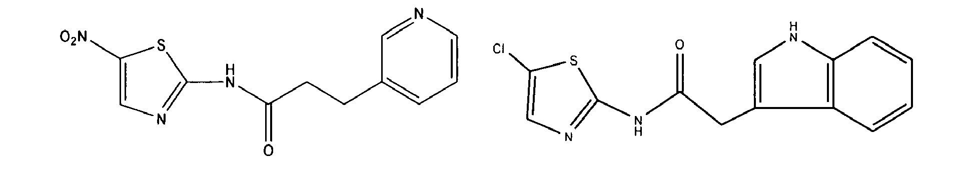

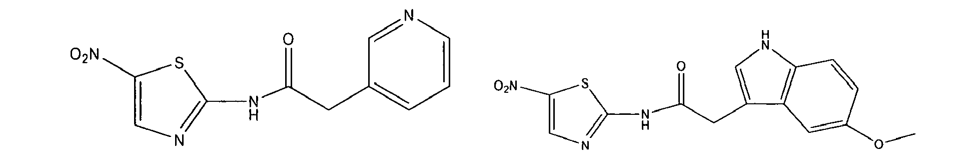

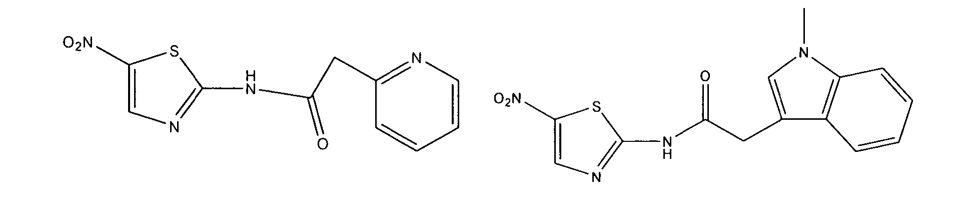

Unless otherwise stated, the novel compounds of formula (I) are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates or prodrugs" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts, prodrugs and derivatives can be carried out by methods known in the art.

For instance, pharmaceutically acceptable salts of the novel compounds of formula (I) are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives are those that increase the bioavailability of the compounds of this invention when such novel compounds are administered to a patient (e.g., by allowing an orally administered compound to be more readily absorbed into the blood) or which enhance delivery of the parent compound to a biological compartment (e.g., the brain or lymphatic system) relative to the parent species.

The novel compounds of formula (I) may be in crystalline form either as free compounds or as solvates (e.g. hydrates) and it is intended that both forms are within the scope of the present invention. Methods of solvation are generally known within the art. Suitable solvates are pharmaceutically acceptable solvates. In a particular embodiment the solvate is a hydrate.

The novel compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

The novel compounds represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

The present invention further provides pharmaceutical compositions comprising at least a novel compound of formula (I) of the present invention, or pharmaceutically acceptable salts, prodrugs or stereoisomers thereof with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the novel compounds of formula (I) or compositions of the present invention may be by any suitable method, such as intravenous infusion, oral preparations, and intraperitoneal and intravenous administration. Oral administration is preferred because of the convenience for the patient and the chronic character of many of the diseases to be treated.

Generally an effective administered amount of a novel compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The novel compounds and compositions of this invention may be used with other drugs to provide a combination therapy. The other drugs may form part of the same composition, or be provided as a separate composition for administration at the same time or at different time.

In another aspect, the present invention is referred to a new compound of formula (I) for use as a medicament.

Novel compounds of formula (I) can be obtained by a pathway strategy which comprises coupling the conveniently pyridyl-, indazolyl- or indolyl-acid of formulas (II), (III) and (IV), respectively: 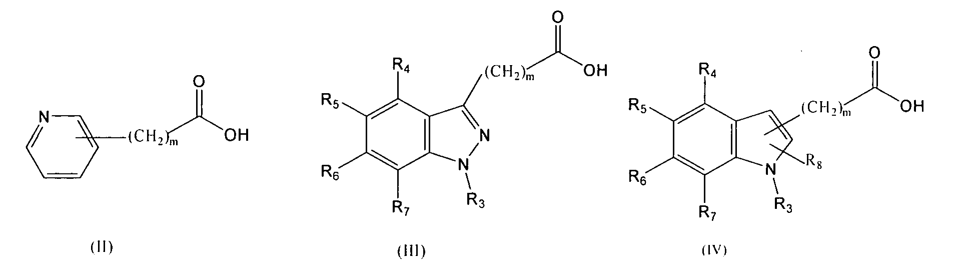 wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl, and
m is an integer from 0 to 6;
with a thiazol of formula (V): 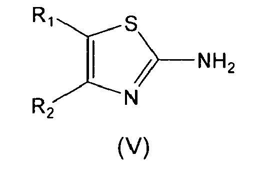 wherein R₁ and R₂ are independently selected from H, -NO₂, halogen, -NH₂ and -CN, with the proviso that at least one of R₁ and R₂ is different from H.

Compounds of formula (II), (III), (IV) and (V) are all commercially available.

### General procedure for the group of compounds wherein X = pyridin

In a particular embodiment of the invention, the compound of formula (I) wherein X is a pyridine, is obtained according to the following general procedure. To a solution of the corresponding pyridyl-acid of formula (II) in anhydrous tetrahydrofurane (THF hereinafter), 1.5 equivalents of N,N'-carbonyldiimidazole (DCI hereinafter) in anhydrous THF are added as activating reagent. The resulting mixture is allowed to stir at room temperature for about 4 to 5 h. Then 1 equivalent of the corresponding thiazol of formula (V) in THF is added to the reaction mixture, and this is stirred at room temperature for about 8 to 10 hours. When the reaction is completed, the solvent is evaporated and the resulting crude is dissolved in CH₂Cl₂ and washed with water. The purification is performed according to general methods of purification known by the Expert.

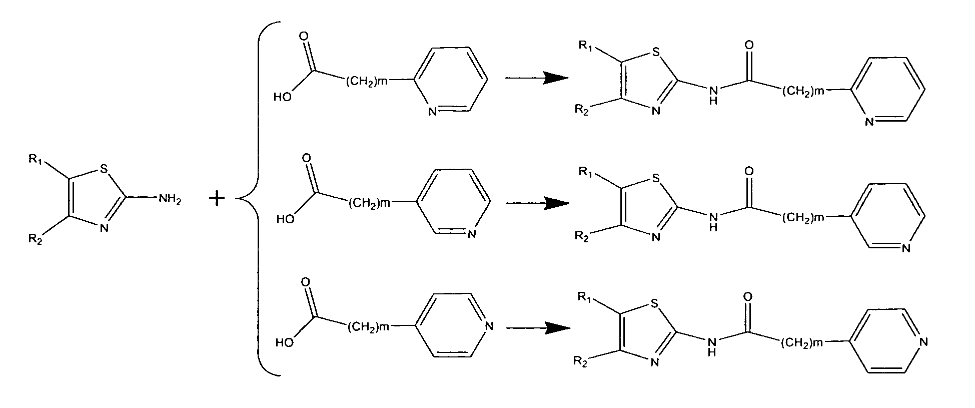

### General procedure for the group of compounds wherein X = indazol

In another particular embodiment of the invention, the compound of formula (I) wherein X is indazole, is obtained according to the following general procedure. To a solution of the corresponding indazole-acid of formula (III) in anhydrous THF, 1.5 equivalents of CDI in anhydrous THF are added as activating reagent. The resulting mixture is allowed to stir at room temperature for about 4 to 5 hours. Then 1 equivalent of the corresponding thiazol of formula (V) in THF is added to the reaction mixture and this is stirred at room temperature for about 8 to 10 hours. When the reaction is completed, the solvent is evaporated and the resulting crude is dissolved in CH₂Cl₂ and washed with water. The purification is performed according to general methods of purification known by the Expert.

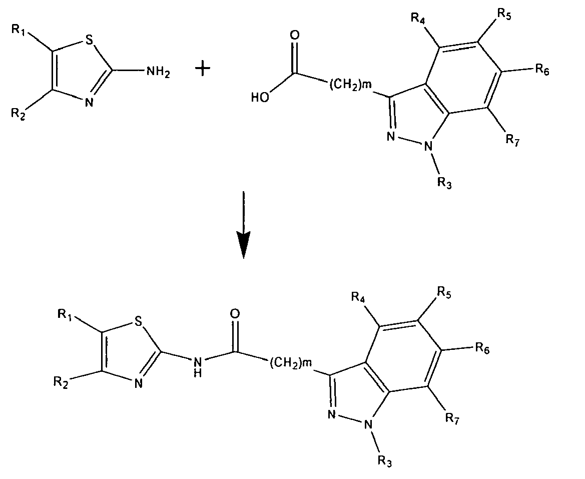

### General procedure for the group of compounds wherein X = indol:

In a further particular embodiment of the invention, the compound of formula (I) wherein X is indol, is obtained according to the following general procedure. To a solution of the indole-acid of formula (IV) in anhydrous THF 1.5 equivalents of CDI in THF anhydrous are added as activating reagent. The resulting mixture is allowed to stir at room temperature for about 4 to 5 h. Then 1 equivalent of the corresponding thiazol of formula (V) in THF is added to the reaction mixture and this is stirred at room temperature for about 8 to 10 hours. When the reaction is finished the solvent is evaporated and the resulting crude is dissolved in CH₂Cl₂ and washed with water. The purification is performed according to general methods of purification known to the Expert.

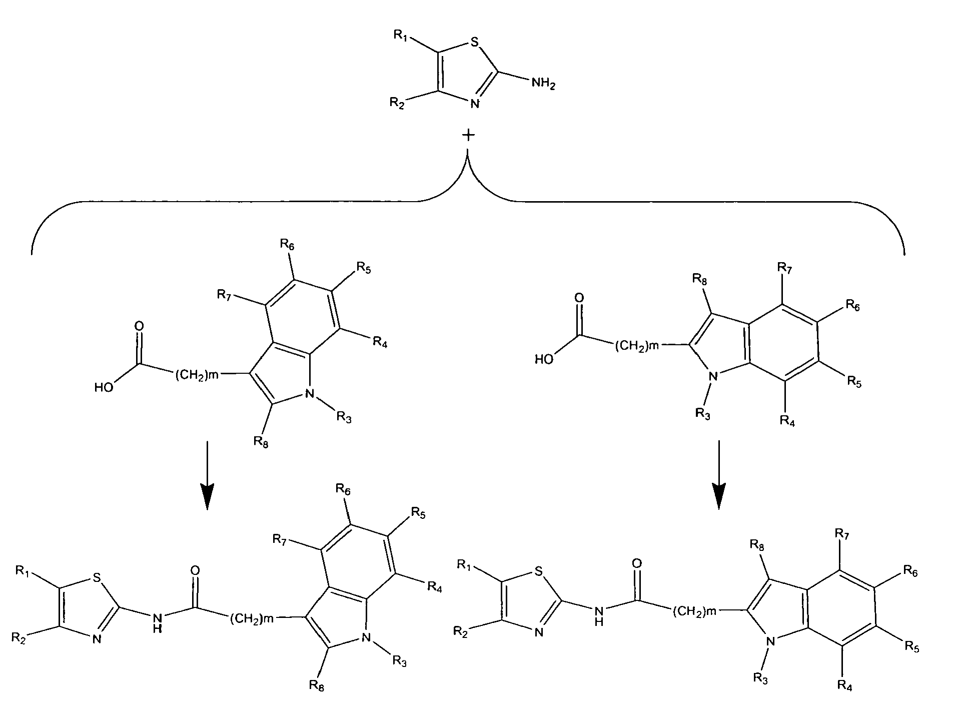

The following examples are given as further illustration of the invention, they should in no case be taken as a definition of the limits of the invention.

### EXAMPLES

### Preparative Examples

In the following, a detailed description of the preparation of some compounds of Formula (I) according to the present invention is given.

### Example 1

### Preparation of N-(5-Nitro-thiazol-2-yl)-2-pyridin-3-yl-acetamide (compound 13)

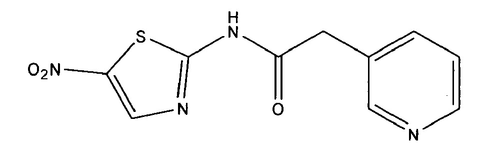

To a solution of 3-Pyridylacetic acid hydrochloride (2.076 g, 12 mmol) in anhydrous THF, 1.5 equivalents of CDI (18 mmol, 2.916 g) in anhydrous THF and 1 equivalent of NEt₃ (1.66 mL) are added. The resulting mixture is allowed to stir at room temperature for 4 hours. Then, 2-amino-5-nitro-thiazol (12 mmol, 1.740 g) in THF is added to the reaction mixture and this is stirred at room temperature for 10 h. When the reaction is completed, the solvent is evaporated and the resulting brown crude is dissolved in CH₂Cl₂ and water. This mixture produces a yellow precipitate, which is filtered and washed with water to obtain the desired compound as a yellow solid (2.300 g, yield: 73 %, 265 M⁺).

¹H-NMR (DMSO): 3.95 (s, 2H); 7.38 (dd, 1H); 7.74 (d, 1H); 8.50 (d, 1H); 8.52 (s, 1H); 8.63 (s, 1H)

¹³C-NMR (DMSO): 38.52; 123.4; 129.7; 137.1; 141.7; 142.6; 148.1; 150.3; 161.8; 170.7

### Example 2

### Preparation of 1H-Indazole-3-carboxylic acid (5-nitro-thiazol-2-yl)-amide (compound 11)

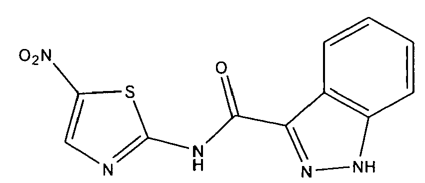

To a solution of 3-indazole acid (3 mmol, 486 mg) in anhydrous THF and 1ml of anhydrous dimethylformamide (DMF) to increase the solubility of the indazole, 1.5 equivalents of CDI (4.5 mmol, 729 mg) in anhydrous THF are added. The resulting mixture is allowed to stir at room temperature for 4,5 hours. Then, 2-amino-5-nitro-thiazol (3 mmol, 435 mg) in THF is added to the reaction mixture and this is stirred at room temperature for 10 h. When the reaction is completed, the solvent is evaporated and the resulting crude is dissolved in CH₂Cl₂ and washed with water (3 x 10 mL). The organic layer is dried with Na₂SO₄. Then, the solvent is removed in vacuum and the residue provided is washed with a mixture of CH₂Cl₂/MeOH to obtain the desired compound as a yellow solid (550 mg, 63%, 290 M⁺).
¹H-NMR (DMSO): 7.37 (t, 1H); 7.51 (t, 1H); 7.72 (d, 1H); 8.21 (d, 1H); 8.67 (s, 1H)
¹³C-NMR (DMSO): 107.9; 111.2; 120.8; 122.0; 123.4; 127.1; 135.5; 141.2; 141.8; 142.7; 161.7; 161.8

### Example 3

### Preparation of 2-(Indol-3-yl)-N-(5-nitro-thiazol-2-yl)-acetamide (compound 4)

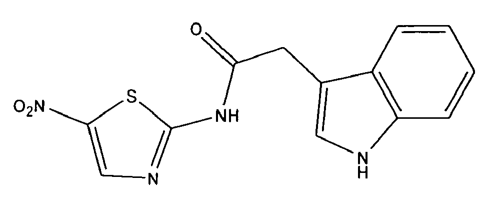

To a solution of 3-indole-acetic acid (2.10 g, 12 mmol) in anhydrous THF, 1.5 equivalents of CDI (18 mmol, 2.916 g) in anhydrous THF are added. The resulting mixture is allowed to stir at room temperature for 5 h. Then, 2-amino-5-nitro-thiazol (12 mmol, 1.740 g) in THF is added to the reaction mixture and this is stirred at room temperature for 10 h. When the reaction is completed, the solvent is evaporated and the resulting brown crude is dissolved in CH₂Cl₂ and washed with water (3 x 30 mL). The organic layer is dried with Na₂SO₄. Then, the solvent is removed in vacuum and the obtained residue is washed with a mixture of CH₂Cl₂/MeOH to obtain the desired compound as a yellow solid (2.260 g, 62%, 303 M⁺).
¹H-NMR (DMSO): 3.96 (s, 2H); 6.97-7.5 (m, 5H); 8.6 (s, 1 H); 10.9 (NH); 13.26 (NH)
¹³C-NMR (DMSO): 32.1; 106.4; 111.4; 118.4; 118.6; 121.1; 124.4; 126.9; 136.0; 141.7; 142.6; 161.9; 171.5

### Example 4

### Preparation of N-(5-Chloro-thiazol-2-yl)-2-(indol-2-yl)-acetamide (compound 6)

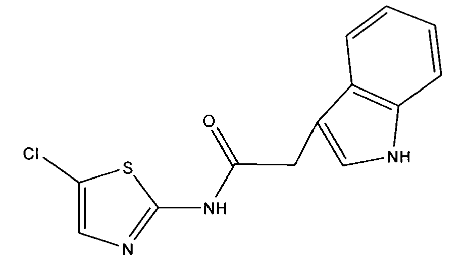

To a solution of 3-indole-acetic acid (525.5 mg, 3mmol) in anhydrous THF, 1.5 equivalents of CDI (4.5 mmol, 729 mg) in anhydrous THF are added. The resulting mixture is allowed to stir at room temperature for 5 h. Then, 2-amino-5-chloro -thiazol (3 mmol, 513.5 mg) with 1 equivalent of NEt₃ (0.41 mL) in THF is added to the reaction mixture and this is stirred at room temperature for 10 h. When the reaction is completed, the solvent is evaporated and the resulting red oil crude is dissolved in CH₂Cl₂ and washed with water (3 x 30 mL). The organic layer is dried with Na₂SO₄. Then, the solvent is removed in vacuum and the residue is purified by silica gel column chromatography CH₂Cl₂/ MeOH; 30/1, to obtain the desired compound as a white solid (500 mg, Yield: 57 %, 293 M+, 290 M-).
¹H-NMR (DMSO): 3.92 (s, 2H); 6.97 (m, 1H); 7.08 (m, 1H); 7.25 (d, 1 H); 7.36 (m, 1H); 7.50 (s, 1H); 7.56 (d, 1H); 8.6 (s, 1H); 10.9 (NH); 12.56 (NH)
¹³C-NMR (DMSO): 31.85; 107.05; 111.38; 117.88; 118.45; 118.51; 121.06; 124.21; 126.96; 135.51; 135.99; 156.00; 170.20

### Example 5

### Preparation of 2-(5-Methoxy-indol-3-yl)-N-(5-nitro-thiazol-2-yl)-acetamide (compound 7)

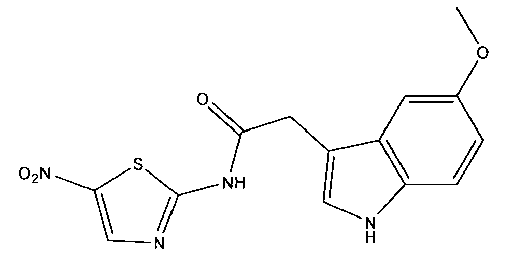

To a solution of (5-Methoxy-indol-3-yl)-acetic acid (410 mg, 2 mmol) in anhydrous THF, 1.5 equivalents of CDI (3 mmol, 486 mg) in anhydrous THF are added. The resulting mixture is allowed to stir at room temperature for 5 h. Then, 2-amino-5-nitro-thiazol (2 mmol, 290 mg) in THF is added to the reaction mixture and this is stirred at room temperature for 10 h. When the reaction is completed, the solvent is evaporated and the resulting brown crude is dissolved in CH₂Cl₂ and washed with water (3 x 30 mL). The organic layer is dried with Na₂SO₄. Then, the solvent is removed in vacuum and the obtained residue is washed with a mixture of CH₂Cl₂/MeOH to obtain the desired compound as a yellow solid (384.5 mg, yield: 50 %, 333 M+).
¹H-NMR (DMSO): 3.73 (s, 3H); 3.95 (s, 2H); 6.75 (dd, 1 H); 7.09 (s, 1H); 7.25 (m, 2H); 8.71 (s, 1H);10.90 (1H, NH)
¹³C-NMR (DMSO): 32.17; 55.33; 100.40; 106.18; 111.15; 112.09; 125.01; 127.31; 131.13; 141.62; 142.71; 153.15; 162.01; 171.60

### Example 6

### Preparation of 2-(1-Methyl-indol-3-yl)-N-(5-nitro-thiazol-2-yl)-acetamide (compound 8)

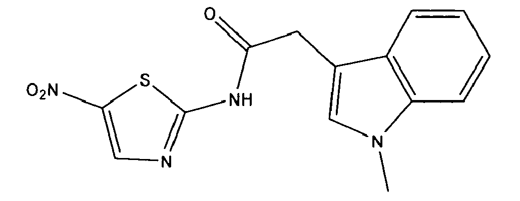

To a solution of (1-Methyl-indol-3-yl)-acetic acid (567.6 mg, 3 mmol) in anhydrous THF, 1.5 equivalents of CDI (4.5 mmol, 729 mg) in anhydrous THF are added. The resulting mixture is allowed to stir at room temperature for 5 h. Then, 2-amino-5-nitro-thiazol (3 mmol, 435 mg) in THF is added to the reaction mixture and this is stirred at room temperature for 10 h. When the reaction is completed, the solvent is evaporated and the resulting brown crude is dissolved in CH₂Cl₂ and washed with water (3 x 30 mL). The organic layer is dried with Na₂SO₄. Then, the solvent is removed in vacuum and the residue is purified by silica gel column chromatography CH2Cl2/MeOH; 40/1, to obtain to obtain the desired compound as a yellow solid (755.5 mg, Yield 80 %; 317M+).
¹H-NMR (DMSO): 3.76 (s, 3H); 3.96 (s, 2H); 7.04 (t, 1H); 7.13 (t, 1H); 7.28 (s, 1H); 7.41 (d, 1H); 7.57 (d, 1H);8.62(s, 1H)
¹³C-NMR (DMSO): 31.80; 32.25; 105.64; 109.61; 118.59; 118.67; 121.20; 127.21; 128.56; 136.39; 141.69; 142.59; 161.78; 171.31

### Example 7

### Preparation of 2-(2-Methyl-indol-3-yl)-N-(5-nitro-thiazol-2-yl)-acetamide (compound 9)

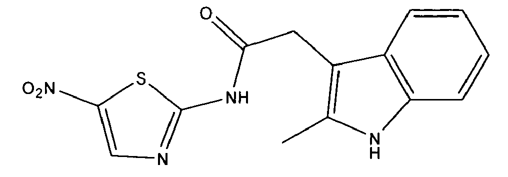

To a solution of (2-Methyl-indol-3-yl)-acetic acid (567.6 mg, 3 mmol) in anhydrous THF, 1.5 equivalents of CDI (4.5 mmol, 729 mg) in anhydrous THF are added. The resulting mixture is allowed to stir at room temperature for 5 h. Then, 2-amino-5-nitro-thiazol (3 mmol, 435 mg) in THF is added to the reaction mixture and this is stirred at room temperature for 10 h. When the reaction is completed, the solvent is evaporated and the resulting brown crude is dissolved in CH₂Cl₂ and washed with water (3 x 30 mL). The organic layer is dried with Na₂SO₄. Then, the solvent is removed in vacuum and the residue is purified by silica gel column chromatography CH2Cl2/MeOH; 40/1, to obtain the desired compound as a yellow solid (726 mg. Yield: 77 %, 317 M+).
¹H-NMR (DMSO): 2.30 (s, 3H); 3.96 (s, 2H); 6.71 (t, 1H); 6.86 (t, 1H); 7.21 (d, 1H); 7.41 (d, 1H); 8.60 (s, 1H);10.90 (1H, NH); 13.21 (1H, NH)
¹³C-NMR (DMSO): 11.35; 30.83; 102.66; 110.35; 117.49; 118.36; 120.13; 128.09; 133.68; 134.96; 141.67; 142.58; 161.79; 171.58

### Example 8

### Preparation of 5-Methoxy-indole-2-carboxylic acid (5-nitro-thiazol-2-yl)-amide (compound 10)

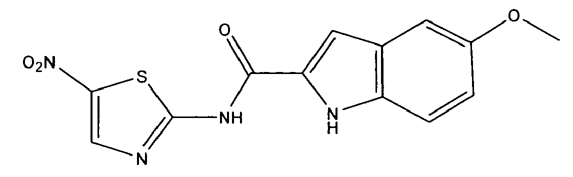

To a solution of 5-Methoxy-indole-2-carboxylic acid (382 mg, 2 mmol) in anhydrous THF, 1.5 equivalents of CDI (3 mmol, 486 mg) in anhydrous THF are added. The resulting mixture is allowed to stir at room temperature for 5 h. Then, 2-amino-5-nitro-thiazol (2 mmol, 290 mg) in THF is added to the reaction mixture and this is stirred at room temperature for 10 h. When the reaction is completed, the solvent is evaporated and the resulting brown crude is dissolved in CH₂Cl₂ and washed with water (3 x 30 mL). The organic layer is dried with Na₂SO₄. Then, the solvent is removed in vacuum and the obtained residue is purified by silica gel column chromatography CH₂Cl₂/ MeOH; 40/1, to obtain the desired compound as a yellow solid (48 mg. Yield: 5 %, 317 M-).
¹H-NMR (DMSO): 3.79 (s, 3H); 6.95 (dd, 1H); 7.15 (s, 1H); 7.37 (d, 1H); 7.67 (s, 1H); 8.71 (s, 1H); 11.90 (1H, NH); 13.41 (1H, NH)
¹³C-NMR (DMSO): 55.19; 102.08; 107.11; 113.45; 117.11; 127.07; 128.14; 133.27; 141.75; 142.74; 154.07; 159.98; 162.41

### Biological Examples

Compounds obtained in examples 1-8, together with another 9 compounds of formula (I), were subjected to two different assays at different concentrations, in order to determine their biological activity.

### GSK-3β inhibition:

This assay is based on the protocol detailed by Upstate Cat. 14-306, making some slight modifications.

Recombinant human glycogen synthase kinase 3β is assayed in MOPS 11 mM pH7.4, EDTA 0.2 mM, EGTA 1,25 mM, MgCl₂ 26,25 mM and sodium orthovanadate 0.25 mM in the presence of 62.5 µM of Phospho-Glycogen Synthase Peptide-2 (GS-2) (TOCRIS, Cat. 1352), 0.5 µCi γ-³³P-ATP and unlabelled ATP (Sigma, A-9187) at a final concentration of 12.5 µM. After incubation for 30 minutes at 30 °C, aliquots are spotted onto P81 phosphocellulose papers. Filters are washed four times for at least 10 minutes each with 1% phosphoric acid and counted with scintillation cocktail in a scintillation counter (PerkinElmer, Microbeta 1450). The activity of GSK-3 is tested at concentrations of 25 and 50 µM, in the presence of the compounds synthesized according to examples 1 to 8 and in the presence of another 9 compounds of formula (I). The results obtained are indicated in Table I (see below), in the form of percentage of GSK-3 activity.

### Inhibition of tau phosphorylation:

Human neuroblastoma SHSY5Y cells were seeded in the presence of Minimum Essential Medium / Nutrient Mixture F-12. One day later, cells are treated with samples for 18 h at 37°C. After treatment, cultures are washed with phosphate-buffered saline and lysed for 30 min at 4°C in extraction buffer (10 mM Tris-HCl, pH 7.4, 100 mM NaCl, 1 mM EDTA, 2 mM Na₃VO₄, 1% Triton X-100, 10% glycerol, 0.1% SDS, 0.5% Sodium deoxycholate, 1 mM PMSF and a protease inhibitor cocktail (Roche, Cat 1 697 498)).

The quantitative determination of phosphorylated human Tau is made taking aliquots of the cell lysate and using a phosporylation-specific antibody against Tau [pS396] in a sandwich ELISA (Biosource, Cat KHB7031). Tau phosphorylation is estimated by measuring the absorbance at 450 nm in a microtiter plate reader (Cultek, Anthos 2010).

The effect of the synthesized compounds according to examples 1-8 and that of another 9 compounds of formula (I) is determined at different final concentrations, namely 50, 100 and 200 µM. Not all the compounds of formula (I) were tested at all the concentrations. The results are indicated in Table 1 (see below) as "NEG" and "POS", respectively meaning "negative" and "positive"; "NEG" means no tau phosphorylation inhibition was detected at the referred concentration of compound (I); "POS" means that at the referred concentration tau phosphorylation inhibition was detected.

**Table 1**

| **Compound No.** | **Formula** | **% GSK-3 Activity** | | **Tau phosphorylation inhibition in cells** | | |
|---|---|---|---|---|---|---|
| | | **25 µM** | **50 µM** | **50 µM** | **100 µM** | **200 µM** |
| **Compound 1** | 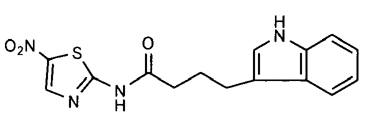 | 37.35 | 18.89 | NEG | NEG | NEG |
| **Compound 2** | 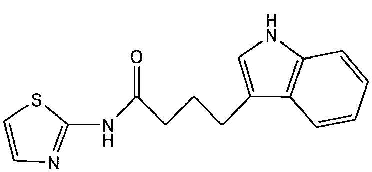 | 59.64 | 38.96 | / | / | / |
| **Compound 3** | 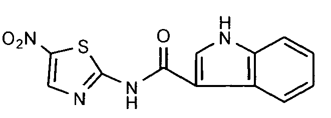 | 73.79 | 53.35 | / | / | / |
| **Compound 4 (Example 3)** | 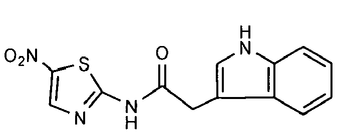 | 6.29 | 8.51 | NEG | POS | POS |
| **Compound 5** | 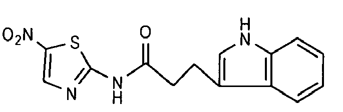 | 19.05 | 12.45 | POS | POS | POS |
| **Compound 6 (Example 4)** | 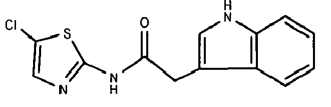 | 29.38 | 14.61 | / | / | / |
| **Compound 7 (Example 5)** | 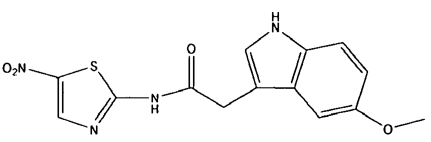 | 43.94 | 32.2 | NEG | POS | / |
| **Compound 8 (Example 6)** | 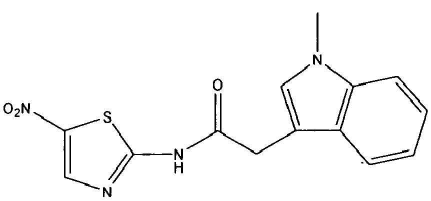 | 17.8 | 9.27 | POS | POS | / |
| **Compound 9 (Example 7)** | 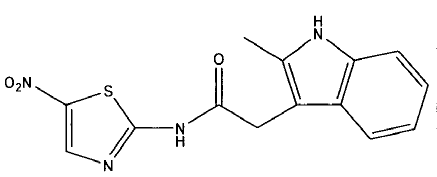 | 7.96 | 2.61 | POS | POS | / |
| **Compound 10 (Example 8)** | 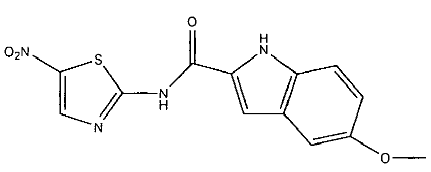 | 18.9 | 10.99 | POS | POS | / |
| **Compound 11 (Example 2)** | 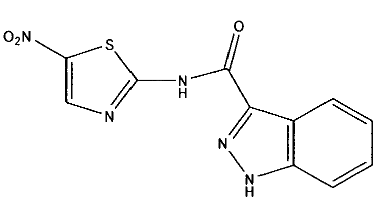 | 34.79 | 18.24 | NEG | NEG | / |
| **Compound 12** | 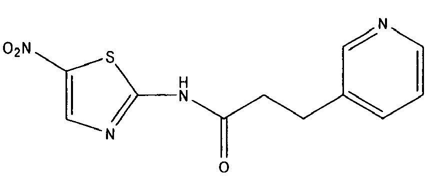 | 8.3 | 5.36 | POS | POS | / |
| **Compound 13 (Example 1)** | 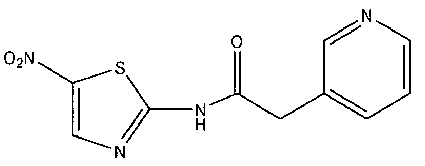 | 5.21 | 2.78 | POS | POS | / |
| **Compound 14** | 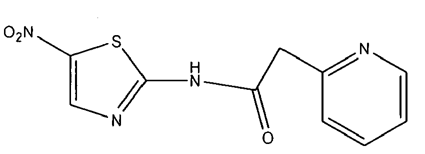 | 9.49 | 4.1 | NEG | POS | / |
| **Compound 15** | 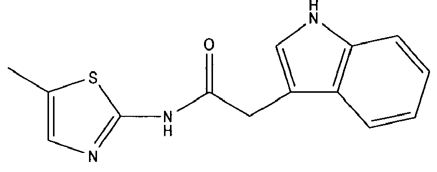 | 49.11 | 37.11 | / | / | / |
| **Compound 16** | 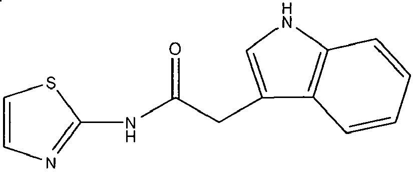 | 70.94 | 42.68 | / | / | / |
| **Compound 17** | 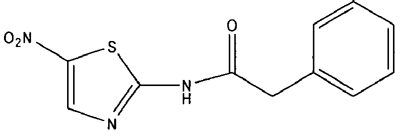 | 13.73 | 8.05 | POS | POS | / |
| **Compound 18** | 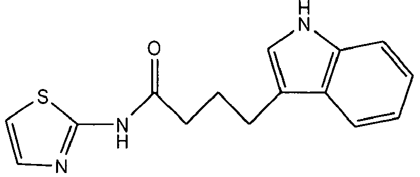 | 59.64 | 38.96 | / | / | / |

In addition to tau phosphorylation assays, quantification of cell death due to potential toxicity of compounds 1, 4, 5, 7, 8, 9, 10, 11, 12, 13 and 14 described above is made by measuring LDH release (Roche, Cat 1 644 793). For the quantitative determination of cell survival, aliquots of the cell lysate are incubated with an equal volume of reaction mixture at room temperature for 20-30 min. The measure of absorbance is made in a microtiter plate reader with 490-492 nm filter (Cultek, Anthos 2010).

For compounds 4, 5, 13 and 14, the cell survival was measured at 24 hours treatment (see Table 2), and for compounds 1, 7, 8, 9, 10, 11 and 12 was measured at 18 hours treatment (see Table 3), in SH-SY5Y cells. It is generally considered that a compound is considered toxic if less than 80% of the cells survive after treatment with a compound.

**Table 2**

| **Compound No.** | **% of cell viability** | | | |
|---|---|---|---|---|
| | 10 µM | 25 µM | 50 µM | 100 µM |
| **Compound 4** | 91.7 ± 1.9 | 90 ± 3.7 | / | / |
| **Compound 5** | 87.8 ± 3.8 | 86.1 ± 8.4 | / | / |
| **Compound 13** | / | / | 94.1 ± 1.8 | 86.5 ± 4.7 |
| **Compound 14** | / | / | 87 ± 6.0 | 85.4 ± 2.7 |

**Table 3**

| **Compound No.** | **% of cell viability** | |
|---|---|---|
| | 50 µM | 100 µM |
| **Compound 1** | 103,2 ± 17 | 101,0 ± 17 |
| **Compound 7** | 99,8 ± 8,6 | 92,9 ± 11,1 |
| **Compound 8** | 103 ± 7,7 | 85,5 ± 6,5 |
| **Compound 9** | 91,6 ± 5,9 | 90,8 ± 5,5 |
| **Compound 10** | 84,7 ± 2,7 | 90,6 ± 7,2 |
| **Compound 11** | 95,3 ± 3,3 | 93,9 ± 5,6 |
| **Compound 12** | 95,7 ± 4,2 | 93,7 ± 7,1 |

In view of the results obtained, the compounds of formula (I) may thus be considered non-toxic.

## Claims

1. Use of a compound of formula (I): 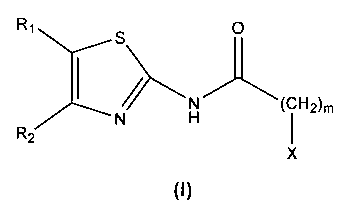 wherein
R₁ and R₂ are independently selected from H, -NO₂, halogen, -NH₂, -CF₃, C₁-C₆ linear alkyl and -CN;
m is an integer from 0 to 6,
X is selected from the group consisting of:
- an indol of formula (A) or (B), bonded respectively at positions 2 or 3: 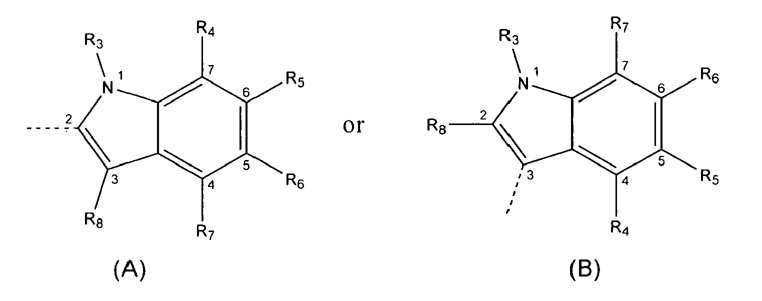
- an indazol of formula (C), bonded at position 3: 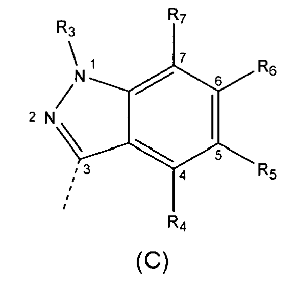
- a pyridin, bonded at any positions 2 to 6; and
- a phenyl ;
wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl,
or any pharmaceutically acceptable salts, solvates and prodrugs thereof, in the preparation of a medicament for the treatment or profilaxis of a disease or condition mediated by GSK-3.

2. Use according to claim 1, wherein the medicament is for the treatment or profilaxis of a disease or condition requiring GSK-3 inhibition.

3. Use according to any one of claims 1 and 2, wherein the disease or condition is selected from diabetes, conditions associated with diabetes, chronic neurodegenerative conditions including dementias such as Alzheimer's disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, Gerstmann-Sträussler-Scheinker disease, Creutzfeld-Jakob disease, prion protein cerebral amyloid angiopathy, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, manic depressive disorder, promotion of functional recovery post stroke, cerebral bleeding (for example, due to solitary cerebral amyloid angiopathy), hair loss, obesity, atherosclerotic cardiovascular disease, hypertension, polycystic ovary syndrome, syndrome X, ischaemia, brain injury, especially traumatic brain injury, cancer, leukopenia, Down's syndrome, Lewy body disease, inflammation, chronic inflammatory diseases, cancer and hyperproliferative diseases as hyperplasias and immunodeficiency.

4. Use according to claim 3, wherein the disease or condition is selected from Alzheimer's disease, diabetes, Parkinson's disease, epilepsy and mood disorders.

5. Use of a compound of formula (I), or any salt or solvate thereof, as reactive for modulating GSK-3 in biological assays, preferably as a reactive for inhibiting GSK-3 activity.

6. A compound of formula (I): 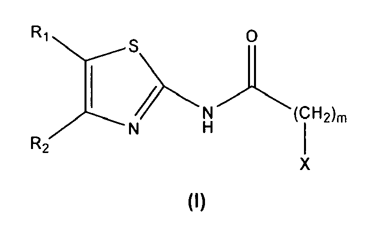 wherein
R₁ and R₂ are independently selected from H, -NO₂, halogen, -NH₂, -CF₃, and
-CN, with the proviso that at least one of R₁ and R₂ is different from H;
m is an integer from 0 to 6,
X is selected from the group consisting of:
- an indol of formula (A) or (B), bonded respectively at positions 2 or 3: 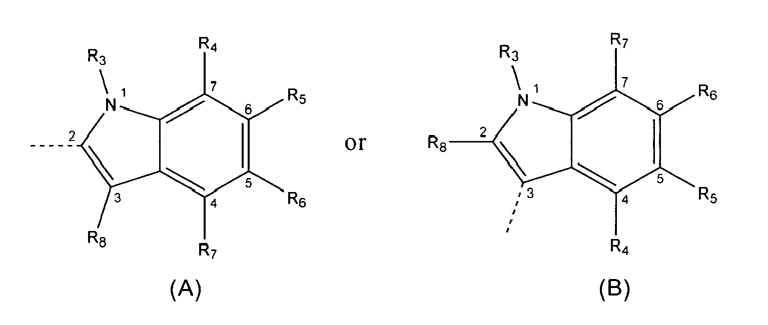
- an indazol of formula (C), bonded at position 3: 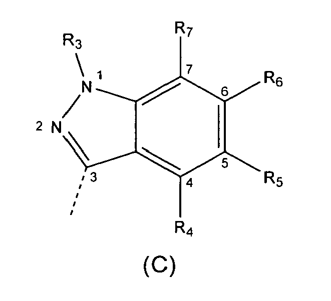 and
- a pyridin, bonded at any positions 2 to 6;
wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl,
or any pharmaceutically acceptable salts, solvates and prodrugs thereof.

7. Compound according to claim 6 wherein X is an indol of formula (A) or (B) as defined in claim 7, bonded respectively at positions 2 or 3.

8. Compound according to claim 6 wherein X is an indazol of formula (C) as defined in claim 7, bonded at position 3.

9. Compound according to any of claims 6 to 8, wherein one of R₁ and R₂ is H.

10. Compound according to any one of claims 6 to 9, wherein one of R₁ and R₂ is -NO₂.

11. Compound according to any one of claims 6 to 10, wherein R₁ is -NO₂ and R₂ is H.

12. Compound according to any one of claims 6 to 11, wherein R₄ to R₇ are independently selected from methoxy and H.

13. Compound according to claim 12 wherein R₄ to R₇ are H.

14. Compound according to any one of claims 6 to 13, wherein R₃ is selected from H and methyl.

15. Compound according to any one of claims 6 to 14, wherein R₈ is selected from H and methyl.

16. Compound according to any of claims 6 to 15, wherein m is 0, 1 or 2.

17. Compound according to claim 6, selected from the following compounds:

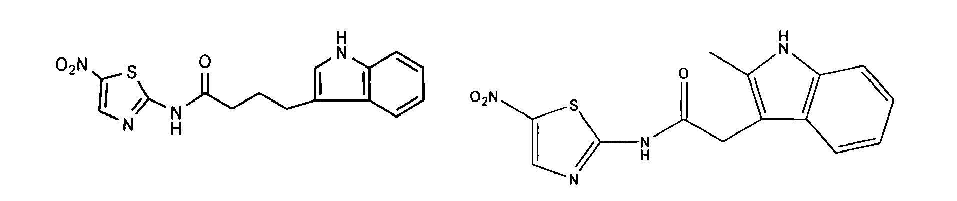

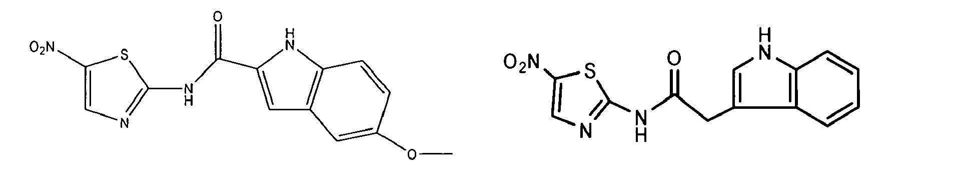

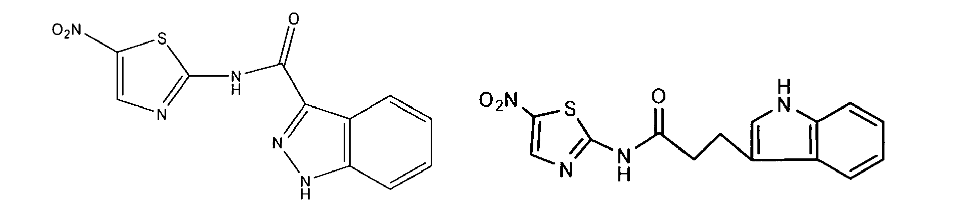

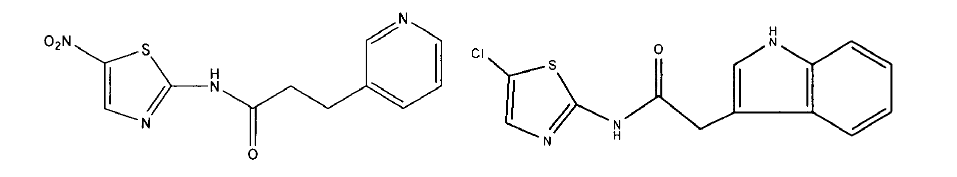

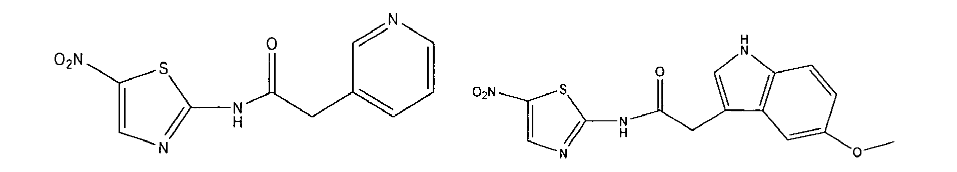

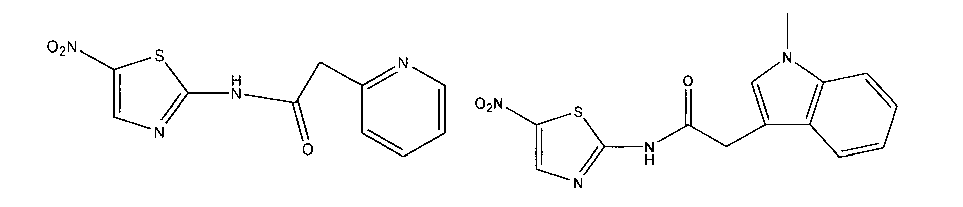

18. Compound according to any one of claims 6 to 17 for use as a medicament.

19. A pharmaceutical composition, comprising at least one compound of formula (I) as defined in any of claims 6 to 17 or a pharmaceutically acceptable salt, prodrug or solvate thereof, and a pharmaceutically acceptable carrier, adjuvant or vehicle.

20. A process for the preparation of a compound of formula (I) as defined in any of claims 6 to 17, comprising coupling a pyridyl-, indazolyl- or indolyl-acid of formula (II), (III) and (IV), respectively: 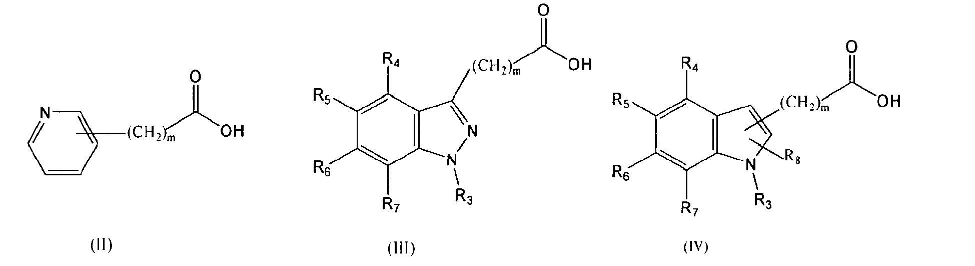 wherein
R₃ is selected from H and C₁-C₆ linear alkyl;
R₄, R₅, R₆ and R₇ are independently selected from H, C₁-C₆ alkyl, C₁-C₆ alkoxy and halogen;
R₈ is selected from H and C₁-C₆ alkyl, and
m is an integer from 1 to 6;
with a thiazol of formula (V): 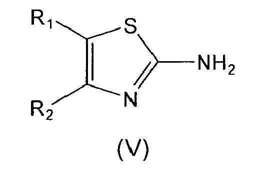 wherein R₁ and R₂ are independently selected from H, -NO₂, halogen, -NH₂, -CF₃, and -CN, with the proviso that at least one of R₁ and R₂ is different from H.
